# EUROPEAN PATENT APPLICATION

(11) **EP 3 332 642 A1**
(43) Date of publication of application: **13.06.2018**
(21) Application number: 16203015.9
(22) Date of filing: 08.12.2016
(51) Int. Cl.: A01N 35/02, A01N 35/04, A01N 31/02, A01P 1/00, A61Q 13/00

(54) **ANTIBACTERIAL PERFUMING COMPOSITION**

(71) Applicant: drom fragrances GmbH & Co. KG, 82065 Baierbrunn (DE)
(72) Inventor: Trinchera, Maria, 82065 Baierbrunn (DE); White, Michael John Robert, 82065 Baierbrunn (DE); Koell, Sandra, 82065 Baierbrunn (DE); Illgen, Astrid, 82065 Baierbrunn (DE)
(74) Representative: dompatent von Kreisler Selting Werner - Partnerschaft von Patent- und Rechtsanwälten mbB

(57) **Abstract**

A composition comprising
a) at least two aldehydes or acetals selected from the group consisting of
4-methoxybenzaldehyde
2-methyl-3-(4-methoxyphenyl)propanal
2-phenylpropanal
6-methoxy-2,6-dimethylheptanal and
1,1-diethoxy-3,7-dimethylocta-2,6-diene
b) at least two alcohols selected from the group consisting of
3,7-dimethyloctane-1,7-diol
2-methyl-1-phenylpropan-2-ol
8,8-dimethoxy-2,6-dimethyloctan-2-ol
3-phenylpropan-1-ol
2-phenylethanol
1-phenylethanol
3,7-dimethyloct-6-en-3-ol and
2-phenylpropan-1-ol.

A personal care, home care, fabric care, air care or fine fragrance product comprising this composition. The use of this composition as an antibacterial component in such a care product.

## Description

The present invention relates to an antibacterial perfuming composition and its use.

Perfuming compositions are used to give the human body, animals, food, objects, and living-spaces "a pleasant scent". They are today present in a number of different goods in the area of personal care, home care, fabric care, air care or fine fragrances. They include cosmetics, laundry agents, air sanitizers and the like.

Many perfuming compositions are not only used to provide a pleasant scent but also to cover or mask unpleasant odors, so called malodours.

Many malodours have their origin in bacterial activities. Examples of bacteria involved in the development of malodours are for example:
- The characteristic human axillary bad odor comes from some specific molecules such as: (E)-3-methyl-2-hexenoic acid (smells like goat); (S)-3-methyl-3-sulfanylhexan-1-ol (onion smell); 3-hydroxy-3-methylhexanoic acid (cumin).
- These molecules are formed by bacterial action on odor precursors that originate from apocrine sweat glands. The permanent population of microorganisms found in the axilla region consists mainly of Gram-positive bacteria of the genera Staphylococcus, Micrococcus, Corynebacterium and Propionibacterium, but many studies suggest that members of the Corynebacterium genus are the primary trigger agents of axillary odor, followed from Staphylococcus epidermis.
- The typical cheese smell of feet is due to the presence of isovaleric acid (cheesy, fermented, rancid odor). Brevibacterium epidermidis is one of the main responsible bacteria for the development of the typical feet malodor, especially when there is high percentage of humidity.
- Body malodors are developed when the odorless perspiration is in contact with Staphylococci and Corynebacteria, which have been identified in many parts of the human body.
- Clothing textiles are in close contact with the microorganisms of the skin and those of the environment. Some fabrics create a warm and often moist environment on the skin, which leads to the growth of bacteria. In some cases, these microorganisms lead to unpleasant odors, staining, fabric deterioration and even physical irritation, like skin allergies and skin infections. Skin microorganisms transfer to the clothing fibers and interact with them. An important factor determining bacteria-fiber interaction is the origin and the composition of textile fibers. A large discrepancy exists in the way bacteria adhere to natural versus synthetic fibers. Some studies show that Micrococci are isolated mainly on synthetic fabrics, while Staphylococci are abundant on both cotton and synthetic fabrics. These microorganisms are the main responsible for generating malodor in fabrics.
- An unpleasant odor is often generated from laundry that is not dried properly. The major microorganism associated with such malodor (mainly due to 4-methyl-3-hexenoic acid) is Moraxella.
- Besides the malodors development, other manifestation attributed to a bacterial origin are skin allergies or skin infection such as, for example, acne which is commonly caused by the presence of Propionibacterium acnes bacteria.

EP 1 181 866 discloses an antibacterial compound named Sandela that combines antibacterial properties with perfume properties.

In general, highly effective preservative or antibacterial ingredients are known but many of them, used especially in cosmetic field, are not well tolerated producing incompatibility reaction or discomfort for the consumer or are strongly questioned by consumers organizations; such as chlorinated compounds (2,4,4'-trichloro-2'-hydroxy-diphenyl-ether), Parabens, Methylisothiazolinone (MIT or MI) and Methylchloroisothiazolinone (CMIT or CMI) or the like. This is one of the reasons why there is a considerable interest in the development of alternative preservatives or preservative-free/ self-preserving cosmetics.

There is still a need for further compositions that may be used in the prevention of malodours and/or have preservative/antibacterial properties.

The object of the present invention is to provide compositions which overcome at least some of the drawbacks of prior art. This object is solved by a composition comprising
a) at least one aldehydes or acetal selected from the group consisting of
   4-methoxybenzaldehyde
   2-methyl-3-(4-methoxyphenyl)propanal
   1,1-diethoxy-3,7-dimethylocta-2,6-diene and
   6-methoxy-2,6-dimethylheptanal
b) at least two alcohols selected from the group consisting of
   3,7-dimethyloctane-1,7-diol
   2-methyl-1-phenylpropan-2-ol
   8,8-dimethoxy-2,6-dimethyloctan-2-ol
   3-phenylpropan-1-ol
   2-phenylethanol
   1-phenylethanol
   3,7-dimethyloct-6-en-3-ol and
   2-phenylpropan-1-ol.

Surprisingly, the compositions of the invention are multifunctional:
- they provide a pleasant scent
- they have preservative properties or booster preservative properties of other compounds;
- they have antibacterial properties useful in the prevention of malodours or are useful as antibacterial ingredients for preventing acne.

It is preferred to use not only two aldehydes/acetals in the composition but three or more aldehydes/acetals as a mixture. Also it is preferred do not only use two alcohols but at least three or at least four alcohols in the mixture.

In preferred embodiments, the composition comprises at least two aldehydes/acetals and at least three alcohols, or at least three aldehydes/acetals and at least two alcohols.

In preferred embodiments, the ratio of the amount of the one or more aldehydes/acetals to the amount of the one or more alcohols is from 1:3 to 5:3 (w/w).

In some embodiments the composition comprises additionally one or more solvents. This is especially useful if the composition is used directly for application. The solvent is preferably a solvent that is volatile at room temperature. Suitable solvents include esters and ethers. One example would be esters of citric acid. Other suitable solvents are dipropylene glycol, isopropyl myristate, propylene glycol and mixtures thereof.

A further embodiment of the present invention is a product comprising the composition of the invention. Because the composition of the present invention is highly effective it is typically sufficient to have rather low amounts in the products. A typical range is from 0.1 to 3 % by weight.

Suitable products are personal care products, care products fabric, home care products, air care products or fine fragrance products.

*Personal care products* are consumer products used in personal hygiene or beautification and covers cosmetic products.

*Home care products* refer to all types of cleaning products used in households comprising a variety of products like household cleaners, dishwashing agents, toilet cleaners and machine clear rinse aid.

*Fabric care products* are products used in the cleaning and washing of cloth and other fabrics. It includes laundry agents and the like.

*Air care products* are products used to provide a pleasant smell in the air. They are available in the form of sprays, solutions or devices that evaporate corresponding products.

*Fine fragrances* are perfumes, eau de toilet and the like.

*Cosmetic products* are products intended to be applied to the human body for cleansing, beautifying, promoting attractiveness or altering the appearance without affecting the body structure or function. Cosmetic products are for example body lotions, shampoos, shower gels, cosmetic wipes and deodorants.

In one embodiment, the composition comprises additionally a malodour coverage formulation.

Such malodour coverage formulations comprise typically compounds selected from the group consisting of
- 1-(3,3-dimethylcyclohexyl)ethyl formate
- 3a,6,6,9a-tetramethyl-2,4,5,5a,7,8,9,9b-octahydro-1H-benzo[e][1]benzofuran
- 16-oxacyclohexadecan-1-one
- methyl 3-oxo-2-pentylcyclopentaneacetate
- 1,4-dioxacycloheptadecane-5,17-dione
- triethyl 2-hydroxypropane-1,2,3-tricarboxylate
- 2-ethyl-3-hydroxypyran-4-one
- diphenylmethanone
- 3,7-dimethyloct-6-enenitrile
- 3-(3-propan-2-ylphenyl)butanal
- 3,7-dimethyloctan-3-ol
- (E)-3-methyl-4-(2,6,6-trimethyl-1-cyclohex-2-enyl)but-3-en-2-one
- 3a,4,5,6,7,7a-hexahydro-4,7-methanoinden-6-yl acetate and mixtures thereof.

Preferably, these compounds are present in the following amounts (in weight-%):

| | |
|---|---|
| 0.1 to 5 | 1-(3,3-dimethylcyclohexyl)ethyl formate |
| 0.1 to 1 | 3a,6,6,9a-tetramethyl-2,4,5,5a,7,8,9,9b-octahydro-1H-benzo[e][1]benzofuran |
| 0.1 to 5 | 16-oxacyclohexadecan-1-one |
| 1 to 5 | methyl 3-oxo-2-pentylcyclopentaneacetate |
| 10 to 30 | 1,4-dioxacycloheptadecane-5,17-dione |
| 40 to 80 | triethyl 2-hydroxypropane-1,2,3-tricarboxylate |
| 0.1 to 1 | 2-ethyl-3-hydroxypyran-4-one |
| 0.1 to 5 | diphenylmethanone |
| 0.1 to 5 | 3,7-dimethyloct-6-enenitrile |
| 0.1 to 5 | 3-(3-propan-2-ylphenyl)butanal |
| 0.1 to 5 | 3,7-dimethyloctan-3-ol |
| 0.1 to 1 | (E)-3-methyl-4-(2,6,6-trimethyl-1-cyclohex-2-enyl)but-3-en-2-one |
| 0.1 to 1 | 3a,4,5,6,7,7a-hexahydro-4,7-methanoinden-6-yl acetate |

A further embodiment of the invention is the use of this product for malodour coverage and malodour prevention.

A further embodiment of the invention is personal care, home care, fabric care, air care or industrial care product comprising the composition of the invention. Typically, the amount of the composition of the invention is from 0.1 to 3% by weight (excluding any solvents in the calculation).

A further embodiment of the invention is a composition comprising a further antibacterial composition. The product of the present invention thus works as a "booster" to known antibacterial compositions to improve their properties. A further embodiment is the use of the composition of the invention as an antibacterial component in a personal care, home care, fabric care, air care or fine fragrance product.

Preferably, the composition is effective against one or more of Staphylococcus Aureus, Pseudomonas aeruginosa, Escherichia coli, Candida albicans, Aspergillus brasiliensis, Corynebacterium Xerosis, Propionibacterium Acnes, Brevibacterium epidermidis, Staphylococcus Epidermidis, Moraxella Species and Micrococci.

### Examples

### Example 1 - Antimicrobial action of single ingredients in a base body lotion

These microbiological challenge tests according to Pharm EUR. Ch 5.1.3. are required for testing preservative efficacy.

Methods for efficacy testing of preservatives acc Pharm. EUR. (challenge test)
Quality requirements
Requirements for sufficient preservation
Inocculated germs are:
   ▪ Staphylocuccus Aureus (SA) (gram pos. bacteria)
   ▪ Pseudomonas Aeruginosa (PA) (gram neg. bacteria)
   ▪ Escherichia Coli (EC) (gram neg. bacteria)
   ▪ Candida Albicans (CA) (yeast)
   ▪ Aspergillus brasiliensis (AB) (mould).

| | Criteria | 2 days | 7 days | 14 days | 28 days |
|---|---|---|---|---|---|
| SA, PA, EC | A | -2 log (99%) | -3 log (99,9%) | | No growth |
| SA, PA, EC | B | | | -3 log (99,9%) | No growth |
| CA, AB | A | | | -2 log (99%) | No growth |
| CA, AB | B | | | -1 log (90%) | No growth |

A number of ingredients were tested in a concentration of 0.4 % in a body lotion on their effect on 5 different microorganisms. The microbial development was followed for 28 days. While some of the ingredients showed good properties against Staphylococcus aureus, Pseudomonas aeruginosa and Escherichia coli most of them showed poor properties against Candida albicans and Aspergillus brasiliensis.

Samples were inoculated with the respective microorganism according to PHARM EUR 2014: 5.1.3.

| ING. | Staph. Aureus | Pseud. Aerugin. | E. Coli | Cand. Albic. | Asp. Bras. |
|---|---|---|---|---|---|
| Farnesol P | B | A | A | - | - |
| 3,7-dimethyloct-6-en-3-ol | A | A | A | - | - |
| 2-phenylpropan-1-ol | A | A | A | - | - |
| 3-phenylpropan-1-ol | A | A | A | - | - |
| MPG/2 Phenoxy Ethanol | A | A | A | - | - |
| 2-methyl-1-phenylpropan-2-ol | B | A | A | - | - |
| TERPINEN-4-OL | B | A | B | - | - |
| DIETHYL PHTHALATE/PALATINOL A (R) | B | A | A | - | - |
| 3,7-DIMETHYLOCTANE-1,7-DIOL | A | A | A | - | - |
| LYRAL | A | A | A | A | - |
| 8,8-dim ethoxy-2,6-dimethyloctan-2-ol | A | A | A | B | - |
| 2-phenylethanol | A | A | A | - | - |
| Triethyl citrate | B | A | B | - | - |
| Hydratropaldehyde | A | A | A | A | - |
| Nerol | A | A | A | - | - |
| 6-methoxy-2,6-dimethylheptanal | A | A | A | B | - |
| 2-methyl-3-(4-methoxyphenyl)propanal | A | A | A | B | - |

Typically, single ingredients may not be used in elevated amounts in a preparation because they may have unpleasant odor or may have side effects, therefore mixtures were tested.

### Example 2 - Testing of a mixture of different alcohols

Three mixtures, with a soft and pleasant smell, were prepared and the preservative efficacy was tested in the same base and at the same dosage used for single ingredients. The results of the mixtures with a soft smell, shown the same efficacy of single ingredients against gram negative bacteria. The efficacy against gram positive does not appear significantly better.

| PRS_13 | |
|---|---|
| name | dosage % |
| Farnesol P | 10 |
| 2-phenylpropan-1-ol | 10 |
| 3,7-dimethyloctane-1,7-diol | 10 |
| 2-methyl-1-phenylpropan-2-ol | 30 |
| DIETHYL PHTHALATE/PALATINOL A (R) | 40 |
| | 100 |

| PRS_14 | |
|---|---|
| name | dosage % |
| MPG /2 Phenoxy Ethanol | 30 |
| 2-PHENYLPROPAN-1-OL | 5 |
| 3,7-DIMETHYLOCTANE-1,7-DIOL | 5 |
| 2-methyl-1-phenylpropan-2-ol | 30 |
| DIETHYL PHTALATE/PALATINOL A (R) | 30 |
| | 100 |

| PRS_15 | |
|---|---|
| name | dosage % |
| MPG /2 Phenoxy Ethanol | 20 |
| 2-PHENYLPROPAN-1-OL | 10 |
| Triethyl citrate | 20 |
| 2-methyl-1-phenylpropan-2-ol | 20 |
| DIETHYL PHTALATE/PALATINOL A (R) | 30 |
| | 100 |

Tests were conducted as in example 1.

| ING. | Staph. Aureus | Pseud. Aeruqin. | E.Coli | Cand. Albic | Asp. Bras. |
|---|---|---|---|---|---|
| PRS_13 | B | A | A | - | - |
| PRS_14 | A | A | A | - | - |
| PRS_15 | B | A | A | - | - |

### Example 3 - Effect of mixtures

Further mixtures were tested in order to increase the efficacy against Staphylococcus Aureus. The analyzed mixtures comprise both aldehydes and alcohols.

| PRS_24 | |
|---|---|
| name | dosage % |
| 2-methyl-3-(4-methoxyphenyl)propanal | 40 |
| 2-methyl-1-phenylpropan-2-ol | 40 |
| 3,7-dimethyloctane-1,7-diol | 7 |
| 8,8-dimethoxy-2,6-dimethyloctan-2-ol | 13 |
| | 100 |

| PRS_28 | |
|---|---|
| name | dosage % |
| 2-methyl-3-(4-methoxyphenyl)propanal | 40 |
| 2-methyl-1-phenylpropan-2-ol | 30 |
| 6-methoxy-2,6-dimethylheptanal | 3 |
| 3,7-dimethyloctane-1,7-diol | 7 |
| 8,8-dimethoxy-2,6-dimethyloctan-2-ol | 20 |
| | 100 |

| PRS_29 | |
|---|---|
| name | dosage % |
| 2-methyl-3-(4-methoxyphenyl)propanal | 35 |
| 2-methyl-1-phenylpropan-2-ol | 25 |
| 3,7-dimethyloctane-1,7-diol | 15 |
| 8,8-dimethoxy-2,6-dimethyloctan-2-ol | 25 |
| | 100 |

As in the examples before, the effects on different bacteria were tested. It can be seen that they have improved efficiency against Staphylococcus Aureus compared to the examples of experiment 2.

Tests were conducted as in example 1.

| ING. | Staph. Aureus | Pseud. Aerugin. | E.Coli | Cand. Albic | Asp. Bras. |
|---|---|---|---|---|---|
| PRS_24 | A | A | A | - | - |
| PRS_28 | A | A | A | - | - |
| PRS_29 | A | A | A | - | - |

### Example 4: Further compositions

Further compositions were prepared and tested in different compositions at different dosages in order to test the efficacy against yeast and mould species.

| PRS_39 | |
|---|---|
| name | dosage % |
| 2-methyl-3-(4-methoxyphenyl)propanal | 10 |
| 4-methoxybenzaldehyde | 31 |
| 2-methyl-1-phenylpropan-2-ol | 5 |
| Triethyl Citrate | 38 |
| 3-phenylpropan-1-ol | 3 |
| 1-phenylethanol | 6 |
| 1,1-diethoxy-3,7-dimethylocta-2,6-diene | 3 |
| 3,7-dimethyloctane-1,7-diol | 1 |
| 8,8-dimethoxy-2,6-dimethyloctan-2-ol | 3 |
| | 100 |

| PRS_40 | |
|---|---|
| name | dosage % |
| 2-methyl-3-(4-methoxyphenyl)propanal | 29 |
| 4-methoxybenzaldehyde | 8 |
| 2-methyl-1-phenylpropan-2-ol | 5 |
| Triethyl Citrate | 35 |
| 3-phenylpropan-1-ol | 3 |
| 1-phenylethanol | 10 |
| 1,1-diethoxy-3,7-dimethylocta-2,6-diene | 6 |
| 3,7-dimethyloctane-1,7-diol | 1 |
| 8,8-dimethoxy-2,6-dimethyloctan-2-ol | 3 |
| | 100 |

| PRS_44 | |
|---|---|
| name | dosage % |
| 2-methyl-3-(4-methoxyphenyl)propanal | 3 |
| 4-methoxybenzaldehyde | 3 |
| 2-methyl-1-phenylpropan-2-ol | 20 |
| Triethyl Citrate | 58 |
| 2-phenylethanol | 7 |
| 3-phenylpropan-1-ol | 3 |
| 1-phenylethanol | 6 |
| | 100 |

| PRS_50 | |
|---|---|
| name | dosage % |
| 2-methyl-3-(4-methoxyphenyl)propanal | 37 |
| 2-methyl-1-phenylpropan-2-ol | 5 |
| Triethyl Citrate | 35 |
| 3-phenylpropan-1-ol | 3 |
| 1-phenylethanol | 10 |
| 1,1-diethoxy-3,7-dimethylocta-2,6-diene | 6 |
| 3,7-dimethyloctane-1,7-diol | 1 |
| 8,8-dimethoxy-2,6-dimethyloctan-2-ol | 3 |
| | 100 |

| PRS_51 | |
|---|---|
| name | dosage % |
| 2-methyl-3-(4-methoxyphenyl)propanal | 12 |
| 2-methyl-1-phenylpropan-2-ol | 20 |
| Triethyl Citrate | 52 |
| 2-phenylethanol | 7 |
| 3-phenylpropan-1-ol | 3 |
| 1-phenylethanol | 6 |
| | 100 |

These products were tested in similar way as in example 1, but at higher concentration.

| Dosage % in body lotion without preservative and perfume (total 5,6%) | | | |
|---|---|---|---|
| | Ing 1 | con. Ing 1 % | water to add |
| Sample 1 | PRS_40 | 1,5 | 4,1 |
| Sample 2 | PRS_44 | 1.5 | 4.1 |
| Sample 3 | PRS_24 | 1.5 | 4.1 |
| Sample 4 | PRS_39 | 0.8 | 4.8 |
| Sample 5 | PRS_50 | 1.5 | 4.1 |
| Sample 6 | PRS_51 | 1.5 | 4.1 |

| ING. | Staph. Aureus | Pseud. Aerugin. | E.Coli | Cand. Albic | Asp. Bras. |
|---|---|---|---|---|---|
| Sample 1 | A | A | A | A | A |
| Sample 2 | A | A | A | - | - |
| Sample 3 | A | A | A | A | - |
| Sample 4 | A | A | A | B | A |
| Sample 5 | A | A | A | A | - |
| Sample 6 | A | A | A | B | - |

### Example 5 - Combination with other preservatives

The above mentioned compositions were tested in combination with other preservatives in a body lotion composition according to the following table:

| Dosage % in body lotion without preservative and perfume (total 5.6%) | | | | | |
|---|---|---|---|---|---|
| | Ing 1 | con. Ing 1 % | Ing 2 | conc. Ing 2 % | water to add |
| Sample 7 | PRS_40 | 0.6 | Phentylene glycol | 5 | 0 |
| Sample 8 | PRS_44 | 0.6 | Phentylene glycol | 5 | 0 |
| Sample 9 | PRS_24 | 0.6 | Phentylene glycol | 5 | 0 |
| Sample 10 | PRS_39 | 0.5 | Phentylene glycol | 5 | 0.1 |
| Sample 11 | PRS_50 | 0.6 | Phentylene glycol | 5 | 0 |
| Sample 12 | PRS_51 | 0.6 | Phentylene glycol | 5 | 0 |
| Sample 13 | PRS_40 | 0.3 | Euxyl PE 9010 | 0.8 | 4.5 |
| Sample 14 | PRS_44 | 0.3 | Euxyl PE 9010 | 0.8 | 4.5 |
| Sample 15 | PRS_24 | 0.3 | Euxyl PE 9010 | 0.8 | 4.5 |
| Sample 16 | PRS_39 | 0.2 | Euxyl PE 9010 | 0.8 | 4.6 |
| Sample 17 | PRS_50 | 0.3 | Euxyl PE 9010 | 0.8 | 4.5 |
| Sample 18 | PRS_51 | 0.3 | Euxyl PE 9010 | 0.8 | 4.5 |
| Sample comparative 19 | Phentylene glycol | 5 | no | | 0.6 |
| Sample comparative 20 | Euxyl PE 9010 | 0.8 | no | | 4.8 |

The compositions gave the following results:

| ING. | Staph. Aureus | Pseud. Aerugin. | E.Coli | Cand. Albic. | Asp. Bras. |
|---|---|---|---|---|---|
| Sample 7 | A | A | A | A | A |
| Sample 8 | A | A | A | A | A |
| Sample 9 | A | A | A | A | A |
| Sample 10 | A | A | A | A | A |
| Sample 11 | A | A | A | A | A |
| Sample 12 | A | A | A | A | A |
| Sample 13 | A | A | A | A | A |
| Sample 14 | A | A | A | A | A |
| Sample 15 | A | A | A | A | B |
| Sample 16 | A | A | A | A | A |
| Sample 17 | A | A | A | A | B |
| Sample 18 | A | A | A | A | B |
| Sample 19 comparative | A | A | A | A | - |
| Sample 20 comparative | A | A | A | A | - |

PRS_40, PRS_50 and PRS_51 were further tested in a shampoo and a shower gel base.

| | | Staph. Aureus | Pseud. Aerugin. | E.Coli | Cand. Albic | Asp. Bras. |
|---|---|---|---|---|---|---|
| Sample A | Shampoo base + PPS_40 at 1.5% | A | A | A | A | A |
| Sample B | Shampoo base + PRS_50 at 1.5% | A | A | A | A | A |
| Sample C | Shampoo base + PRS_51 at 1.5% | A | A | A | A | B |
| Sample E | Shampoo base + PRS_40 at 1.5% | A | A | A | A | A |
| Sample F | Shampoo base + PPS_50 at 1.5% | A | A | A | A | A |
| Sample G | Shampoo base + PRS_51 at 1.5% | A | A | A | A | A |

### Example 6

### Antimicrobial mixtures against specific odor-causing bacteria.

Some of the antimicrobial mixtures was tested against specific odor-causing bacteria in corresponding applications of interest.

| Showergel base + PRS40 at 1.5% | | | | |
|---|---|---|---|---|
| Test strains | | | Incubation time | Total viable counts/g of product |
| Staphylococcus epidermidis | DSM | 1798 | 0 min | 4,06E+05 |
| | | | 10 min | 1,32E+05 |
| | | | 30 min | 1,25E-05 |
| | | | 1 h | 8,09E+04 |
| Corynebacterium xerosis | DSM | 20743 | 0 min | 1,35E+05 |
| | | | 10 min | 1,68E+05 |
| | | | 30 min | 1,69E+04 |
| | | | 1 h | 1,12E+03 |
| Propionibacterium acnes | DSM | 1897 | 0 min | 1,72E+05 |
| | | | 10 min | 4,60E+02 |
| | | | 30 min | 1,00E+02 |
| | | | 1 h | 2,00E+01 |

| Showergel base + PRS50 at 1.5% | | | | |
|---|---|---|---|---|
| Test strains | | | Incubation time | Total viable counts/g of product |
| Staphylococcus epidermidis | DSM | 1798 | 0 min | 4,06E+05 |
| | | | 10 min | 1,57E+05 |
| | | | 30 min | 1,27E+05 |
| | | | 1 h | 1,00E+05 |
| Corynebacterium xerosis | DSM | 20743 | 0 min | 1,35E+05 |
| | | | 10 min | 1,29E+05 |
| | | | 30 min | 2,60E+04 |
| | | | 1 h | 1,52E+03 |
| Propionibacterium acnes | DSM | 1897 | 0 min | 1,72E+05 |
| | | | 10 min | 7,36E+02 |
| | | | 30 min | 5,90E+02 |
| | | | 1 h | 2,00E+01 |

| Showergel base + PRS51 at 1.5% | | | | |
|---|---|---|---|---|
| Test strains | | | Incubation time | Total viable counts/g of product |
| Staphylococcus | DSM | 1798 | 0 min | 4,06E+05 |
| epidermidis | | | 10 min | 1,58E+05 |
| | | | 30 min | 1,56E+05 |
| | | | 1 h | 1,24E+05 |
| Corynebacterium | DSM | 20743 | 0 min | 1,35E+05 |
| xerosis | | | 10 min | 1,63E+05 |
| | | | 30 min | 6,50E+04 |
| | | | 1 h | 1,11E+04 |
| Propionibacterium | DSM | 1897 | 0 min | 1,72E+05 |
| acnes | | | 10 min | 1,86E+03 |
| | | | 30 min | 4,90E+02 |
| | | | 1 h | 8,00E+01 |

| Showerqel base + Benchmark (Sodium Benzoate at 0.5%) | | | | |
|---|---|---|---|---|
| Test strains | | | Incubation time | Total viable counts/g of product |
| Staphylococcus | DSM | 1798 | 0 min | 4,06E+05 |
| epidermidis | | | 10 min | 4,60E+05 |
| | | | 30 min | 4,60E+05 |
| | | | 1 h | 4,00E+05 |
| Corynebacterium | DSM | 20743 | 0 min | 1,35E+05 |
| xerosis | | | 10 min | 2,00E+05 |
| | | | 30 min | 1,40E+05 |
| | | | 1 h | 1,18E+05 |
| Propionibacterium | DSM | 1897 | 0 min | 1,72E+05 |
| acnes | | | 10 min | 4,90E+04 |
| | | | 30 min | 5,65E+04 |
| | | | 1 h | 4,39E+04 |

| BodyLotion base + PRS40 at 1.5% | | | | |
|---|---|---|---|---|
| Test strains | | | Incubation time | Total viable counts/g of product |
| Staphylococcus | DSM | 1798 | 0 min | 4,06E+05 |
| epidermidis | | | 2h | 7,18E+03 |
| | | | 6h | 1,00E+01 |
| Brevibacterium | DSM | 20600 | 0 min | 5,95E+05 |
| epidermidis | | | 2h | 2,00E+01 |
| | | | 6h | <10 |

| BodyLotion base + PRS50 at 1.5% | | | | |
|---|---|---|---|---|
| Test strains | | | Incubation time | Total viable counts/g of product |
| Staphylococcus | DSM | 1798 | 0 min | 4,06E+05 |
| epidermidis | | | 2h | 1,80E+03 |
| | | | 6h | <10 |
| Brevibacterium | DSM | 20600 | 0 min | 5,95E+05 |
| epidermidis | | | 2h | 6,00E+01 |
| | | | 6h | <10 |

| BodyLotion base + PRS51 at 1.5% | | | | |
|---|---|---|---|---|
| Test strains | | | Incubation time | Total viable counts/g of product |
| Staphylococcus | DSM | 1798 | 0 min | 4,06E+05 |
| epidermidis | | | 2h | 2,26E+04 |
| | | | 6h | <10 |
| Brevibacterium | DSM | 20600 | 0 min | 5,95E+05 |
| epidermidis | | | 2h | 2,00E+02 |
| | | | 6h | <10 |

| BodyLotion base + Benchmark Euxyl PE 9010 at 0,8% | | | | |
|---|---|---|---|---|
| Test strains | | | Incubation time | Total viable counts/g of product |
| Staphylococcus | DSM | 1798 | 0 min | 4,06E+05 |
| epidermidis | | | 2h | 1,00E+02 |
| | | | 6h | <10 |
| Brevibacterium | DSM | 20600 | 0 min | 5,95E+05 |
| epidermidis | | | 2h | 1,00E+01 |
| | | | 6h | <10 |

The efficacy of the mixtures in body lotion base against Brevibacterium epidermidis, one of the main responsible bacteria for the feet malodor development is very good.

The antimicrobial mixtures show also effect against bacteria involved in the development of sweat and general body odors like Staphylococcus epidermidis and Corynebacterium xerosis in body lotion and some microbial reduction instead in showergel base.

The mixtures are highly effective when tested in a Showergel base against Propionibacterium acnes which is well known as main responsible bacteria for acne.

### Example 7 - Efficacy of antimicrobial mixtures against Corynebacterium xerosis in deodorant base

The action against the main responsible bacteria for the development of sweat malodour was tested in deodorant base.

| Deodorant base + PRS_40 at 1.5 % | | | | |
|---|---|---|---|---|
| Test strains | | | Incubation time | Total viable counts/g of product |
| Staphylococcus | DSM | 1798 | 0 min | 2.45E+05 |
| epidermidis | | | 1 h | 1.4E+05 |
| | | | 6 h | 2.0E+02 |
| Corynebacterium | DSM | 20743 | 0 min | 2.63+05 |
| xerosis | | | 1 h | 1.0E+03 |
| | | | 6 h | <10 |

| Deodorant base + PRS_50 at 1.5 % | | | | |
|---|---|---|---|---|
| Test strains | | | Incubation time | Total viable counts/g of product |
| Staphylococcus | DSM | 1798 | 0 min | 2.45E+05 |
| epidermidis | | | 1 h | 2.4E+05 |
| | | | 6 h | 8.8E+02 |
| Corynebacterium | DSM | 20743 | 0 min | 2.63E+05 |
| xerosis | | | 1 h | 3.0E+03 |
| | | | 6 h | <10 |

| Deodorant base + PRS_51 at 1.5 % | | | | |
|---|---|---|---|---|
| Test strains | | | Incubation time | Total viable counts/g of product |
| Staphylococcus | DSM | 1798 | 0 min | 2.45E+05 |
| epidermidis | | | 1 h | 2.5E+05 |
| | | | 6 h | 1.4E+05 |
| Corynebacterium | DSM | 20743 | 0 min | 2.63E+05 |
| xerosis | | | 1 h | 2.7E+05 |
| | | | 6 h | <10 |

| Deodorant base only | | | | |
|---|---|---|---|---|
| Test strains | | | Incubation time | Total viable counts/g of product |
| Staphylococcus | DSM | 1798 | 0 min | 2.45E+05 |
| epidermidis | | | 1 h | 3.0E+05 |
| | | | 6 h | 2.8E+05 |
| Corynebacterium | DSM | 20743 | 0 min | 2.63E+05 |
| xerosis | | | 1 h | 2.8E+05 |
| | | | 6 h | 2.1E+05 |

| Deodorant base + 1.5 Sensivia (Benchmark) | | | | |
|---|---|---|---|---|
| Test strains | | | Incubation time | Total viable counts/g of product |
| Staphylococcus | DSM | 1798 | 0 min | 2.45E+05 |
| epidermidis | | | 1 h | 2.9E+05 |
| | | | 6 h | 2.2E+05 |
| Corynebacterium | DSM | 20743 | 0 min | 2.63E+05 |
| xerosis | | | 1 h | 1.4E+05 |
| | | | 6 h | 2.0E+02 |

These experiments show that the compositions of the present invention are highly effective in deodorant preparations and better than the corresponding benchmark products.

### Example 8 - Efficacy of the malodour coverage mixture -antimicrobial mixture against Corynebacterium xerosis in deodorant base.

Some mixtures developed to cover bad odors (MCM-Malodor coverage mixtures) have been added to the AM (antimicrobial mixture) in order to have a combination which could potentially have a double effect: acting from microbiological point of view to prevent the formation of bad odors, and acting against already formed malodorant compounds.

In order to validate the double effect against sweat malodor in deodorant base, it has been carry out a sensorial test to show the effectiveness of MCM in covering sweat malodour, and afterwards an antimicrobial test of the accord ACM-AM against the main responsible bacteria for the development of sweat malodor.

### EFFECTIVENESS OF MCM IN COVERING SWEAT MALODOUR

A cosmetic study was conducted with a 3 days study period per subject. The subjects conducted a 7 day preconditioning period (wash-out) using unscented soap with no other cosmetic products or perfumes.

Two baseline measurements were conducted 24 hours after controlled washing.

Treatment was performed after repeated washing and measurement was 24 hours after single application of the products.

The products were applied for two seconds of spraying, 1 - 1,5 g/axilla. Measurement was performed by a sniffer panel (six trained persons, the panel selected following DIN EN 13725).

Body odor was measured on a scale from 0 (no perspiration odor) to 5 (extreme perspiration odor). Subjects that had a baseline sniff score <2.0 were excluded.

The results were the followings:

| Composition | T0(24h) | T1(24h) | T1-T0 |
|---|---|---|---|
| Original sniff scores (N=27) | | | Difference of Sniff-Scores (N=27) |
| MCM01 | 2.90 | 1.93 | -0.97 |
| MCM02 | 2.8 | 2.03 | -0.77 |
| Difference in Sniff Scores (N=27) | | | |
| 01 vs. 02 | 0.1 | -0.1 | -0.2 |

Results: Treatments resulted in significantly decreased scores compared to baseline

### Composition of MCM01 and MCM02:

| **MCM 01** | % |
|---|---|
| 3a,4,5,6,7,7a-hexahydro-4,7-methanoinden-6-yl acetate | 1.1 |
| 3,7-dimethyloct-6-enenitrile | 0.8 |
| 3-(3-propan-2-ylphenyl)butanal | 1.1 |
| (E)-3-methyl-4-(2,6,6-trimethyl-1-cyclohex-2-enyl)but-3-en-2-one (50% min.) | 0.1 |
| propan-2-ol | 2 |
| 1,4-dioxacycloheptadecane-5,17-dione | 24 |
| 3,7-dimethyloctan-3-ol | 1.1 |
| triethyl 2-hydroxypropane-1,2,3-tricarboxylate | 67.2 |
| diphenylmethanone | 2.6 |
| 100 | |

| **MCM 02** | |
|---|---|
| 1-(3,3-dimethylcyclohexyl)ethyl formate | 1.1 |
| 3a,6,6,9a-tetramethyl-2,4,5,5a,7,8,9,9b-octahydro- 1H-benzor[e][1]benzofuran | 0.1 |
| 16-oxacyclohexadecan-1-one | 1.1 |
| methyl 3-oxo-2-pentylcyclopentaneacetate | 3.3 |
| propan-2-ol | 2 |
| 1,4-dioxacycloheptadecane-5,17-dione | 24 |
| 3,7-dimethyloctan-3-ol | 1.1 |
| triethyl 2-hydroxypropane-1,2,3-tricarboxylate | 67.2 |
| 2-ethyl-3-hydroxypyran-4-one | 0.1 |
| 100 | |

### ANTIMICROBIAL EFFECTIVENESS OF MCM-AM COMBINATION

| PRS_40 at 1.5 % + MCM01 at 1% | | | | |
|---|---|---|---|---|
| Test strains | | | Incubation time | Total viable counts/g of product |
| Staphylococcus | DSM | 1798 | 0 min | 2.45E+05 |
| epidermidis | | | 1 h | 1.7E+05 |
| | | | 6 h | <10 |
| Corynebacterium | DSM | 20743 | 0 min | 2.63+05 |
| xerosis | | | 1 h | <10 |
| | | | 6 h | <10 |

| PRS_50 at 1.5 % + MCM01 at 1% | | | | |
|---|---|---|---|---|
| Test strains | | | Incubation time | Total viable counts/g of product |
| Staphylococcus | DSM | 1798 | 0 min | 2.45E+05 |
| epidermidis | | | 1 h | 1.8E+05 |
| | | | 6 h | <10 |
| Corynebacterium | DSM | 20743 | 0 min | 2.63E+05 |
| xerosis | | | 1 h | <10 |
| | | | 6 h | <10 |

| PRS_51 at 1.5 % + MCM01 at 1% | | | | |
|---|---|---|---|---|
| Test strains | | | Incubation time | Total viable counts/g of product |
| Staphylococcus | DSM | 1798 | 0 min | 2.45E+05 |
| epidermidis | | | 1 h | 2.4E+05 |
| | | | 6 h | 8.2E+01 |
| Corynebacterium | DSM | 20743 | 0 min | 2.63E+05 |
| xerosis | | | 1 h | 1.1E+03 |
| | | | 6 h | <10 |

| + MCM01 at 1% | | | | |
|---|---|---|---|---|
| Test strains | | | Incubation time | Total viable counts/g of product |
| Staphylococcus | DSM | 1798 | 0 min | 2.45E+05 |
| epidermidis | | | 1 h | 2.2E+05 |
| | | | 6 h | 8.1E+04 |
| Corynebacterium | DSM | 20743 | 0 min | 2.63E+05 |
| xerosis | | | 1 h | 2.6E+05 |
| | | | 6 h | 8.0E+03 |

It can be seen that the MCM does not have strong antibacterial properties on its own, but does not interfere with the antibacterial properties of the product of the invention.

## Claims

1. A composition comprising a mixture of
a) at least two aldehydes or acetals selected from the group consisting of
4-methoxybenzaldehyde
2-methyl-3-(4-methoxyphenyl)propanal
1,1-diethoxy-3,7-dimethylocta-2,6-diene and
6-methoxy-2,6-dimethylheptanal
b) at least two alcohols selected from the group consisting of 3,7-dimethyloctane-1,7-diol
2-methyl-1-phenylpropan-2-ol
8,8-dimethoxy-2,6-dimethyloctan-2-ol
3-phenylpropan-1-ol
2-phenylethanol
1-phenylethanol
3,7-dimethyloct-6-en-3-ol and
2-phenylpropan-1-ol.

2. The composition of claim 1 comprising three, four or more aldehydes or acetals selected from a) in claim 1 and four or more alcohols selected from b) in claim 1.

3. The composition of any one of claims 1 to 2, further comprising one or more solvents.

4. The composition of claim 3, wherein the one or more solvents are selected from triethyl citrate, dipropylene glycol isopropyl myristate or propylene glycol and mixtures thereof.

5. The composition of any one of claims 1 to 4, comprising a) to b) in a weight:weight ratio of 1:3 to 5:3.

6. The composition of any one of claims 1 to 5 wherein the composition has antibacterial properties.

7. The composition of any one of claims 1 to 6 wherein the composition comprises additionally a malodour coverage formulation.

8. The composition of 7 wherein the malodour coverage formulation comprises compounds selected from the group consisting of
1-(3,3-dimethylcyclohexyl)ethyl formate
3a,6,6,9a-tetramethyl-2,4,5,5a,7,8,9,9b-octahydro-1H-benzo[e][1]benzofuran
16-oxacyclohexadecan-1-one methyl 3-oxo-2-pentylcyclopentaneacetate
1,4-dioxacycloheptadecane-5,17-dione triethyl 2-hydroxypropane-1,2,3-tricarboxylate
2-ethyl-3-hydroxypyran-4-one diphenylmethanone
3,7-dimethyloctan-3-ol
3,7-dimethyloct-6-enenitrile
3-(3-propan-2-ylphenyl)butanal
(E)-3-methyl-4-(2,6,6-trimethyl-1-cyclohex-2-enyl)but-3-en-2-one
3a,4,5,6,7,7a-hexahydro-4,7-methanoinden-6-yl acetate and mixtures thereof.

9. A personal care, home care, fabric care, air care or fine fragrance product comprising the composition of any one of claims 1 to 8.

10. The product according to claim 9, wherein the amount of the composition is from 0.1 to 3% by weight.

11. The product according to claim 9 or 10, comprising a further antibacterial composition.

12. Use of a composition of any one of claims 1 to 8 as an antibacterial component in a personal care, home care, fabric care, air care or fine fragrance product.

13. Use according to claim 12, wherein the composition is effective against one or more of Staphylococcus Aureus, Pseudomonas aeruginosa, Escherichia coli, Candida albicans, Aspergillus brasiliensis, Corynebacterium Xerosis, Propionibacterium Acnes, Brevibacterium epidermidis, Staphylococcus Epidermidis, Moraxella Species and Micrococci.

14. Use of a composition of any one of claims 1 to 8 as an antibacterial booster for other antibacterial compounds.

15. Use of a composition of any one of claims 7 to 8 for malodour coverage and malodour prevention.
